# EUROPÄISCHE PATENTANMELDUNG

(11) **EP 0 787 986 A1**
(43) Veröffentlichungstag der Anmeldung: **06.08.1997**
(21) Anmeldenummer: 97100409.8
(22) Anmeldetag: 13.01.1997
(51) Int. Cl.: G01N 33/53, G01N 33/543, G01N 33/536, G01N 33/58, G01N 33/68, G01N 33/569

(54) **Verfahren zur Vergrösserung des Assay-Bereiches und zum Vermeiden des Hook-Effektes bei simultanen Sandwich-Immunoassays und entsprechende Testkits**

(30) Priorität: 25.01.1996 DE 19602491
(71) Anmelder: BAYER AG, 51368 Leverkusen (DE)
(72) Erfinder: Köcher, Jürgen, Dr., 40764 Langenfeld (DE)

(57) **Zusammenfassung**

Die vorliegende Erfindung betrifft simultane Sandwich-Immunoassays und Testkits, die affinitätschromatographisch gereinigte polyklonale Antikörper enthalten. Durch die vorgeschaltete affinitätschromatogrpahische Reinigung des polyklonalen Antikörpers wird im Test der Hook-Effekt vermindert und der Analysenbereich erweitert.

## Beschreibung

Zum Nachweis von Proteinen in Serum- und Urinproben für medizinischdiagnostische Zwecke werden häufig wegen besonders guter Spezifität und Sensitivität Immunoassays eingesetzt. Eine besonders wichtige Variante eines Immunoassays stellt der sogenannte Sandwich-Assay dar. Bei diesem Assay binden an zwei verschiedene Epitope des Proteins zwei Antikörper, wobei der eine Antikörper zur Abtrennung von der flüssigen Phase an eine feste Phase gekoppelt wird, beispielsweise an Trägermatierialien wie Mikrotiterplatten aus Polystyrol oder Partikel aus polymeren Teilchen. Der zweite Antikörper dient zum Nachweis des gebildeten Immunkomplexes und ist zu diesem Zweck mit einem Label wie beispielsweise einem Farbstoff, einem Fluoreszenz- oder Chemilumineszenzlabel, einem radioaktiven Nuklid oder einem Enzym gekoppelt.

Es sind zwei Arten von Sandwich-Immunoassays bekannt, die als "Ein-Schritt-" oder simultaner Assay und als "Zwei-Schritt-" oder sequenzieller Immunoassay bekannt sind. Beide Typen unterscheiden sich durch die Reihenfolge der Zugabe der beiden Antikörperreagenzien. Der simultane Immunoassay wird durchgeführt, indem man den Festphasenantikörper (Fängerantikörper) und den signalerzeugenden Antikörper simultan mit der proteinhaltigen Probe mischt, und dann den gebildeten Immunkomplex zur Signalmessung durch Binden an die feste Phase von der flüssigen Phase abtrennt.

Im Gegensatz dazu wird beim sequenziellen oder Zwei-Schritt-Assay zunächst nur der Fängerantikörper mit der proteinhaltigen Probe inkubiert. Der gebildete Antikörper-Antigenkomplex wird an die Festphase gebunden, und das überschüssige Antigen wird durch Waschen entfernt. Erst danach wird im Inkubationsschritt der immobilisierte Antikörper-Antigenkomplex mit dem zweiten gelabeiten Signalantikörper inkubiert.

Der simultane "Ein-Schritt-"Immunoassay gewinnt aufgrund seiner Schnelligkeit der Durchführung gegenüber dem traditionellen "Zwei-Schritt-"Assay immer mehr an Bedeutung. Weiterhin sind Sensitivität und Präzision günstiger.

Ein grundsätzliches Problem bei quantitativen immunologischen Bestimmungsverfahren mit Hilfe des simultanen Sandwich-Immunoassays ergibt sich aus der Form der Reaktionskurve. Von der Konzentration "O" bis zu einer bestimmten Grenzkonzentration des Analyten besitzt die Eichkurve eine positive Steigung. Bei weiterer Erhöhung der Analytkonzentration durchschreitet die Kurve ein Maximum, um danach bei noch höheren Konzentrationen wieder abzufallen. Die Eichkurve besitzt damit für sehr hohe Konzentrationen eine negative Steigung.

Die Analyse einer solchen Eichkurve zeigt, daß einem Meßsignal zwei unterschiedliche Analytkonzentrationen zugeordnet werden können, was wiederum zu einer fehlerhaften Bestimmung des Analyten führen kann. Dieser Effekt wird als "Hook-Effekt" bezeichnet.

Bei der Bestimmung des Analyten durch einen simultanen Sandwich-Immunassay ist es daher erforderlich festzustellen, ob sich das Meßsignal im aufsteigenden oder absteigenden Ast der Kurve befindet, um eine fehlerhafte Bestimmung aufgrund dieses Hook-Effektes zu vermeiden.

Im Bereich der positiven Steigung der Eichkurve für niedrigere Konzentrationen ergibt sich eine weitere Schwierigkeit dadurch, daß bei Annäherung an den Hook-Peak die Kurve so flach verläuft, daß eine Diskriminierung von nah beieinanderiiegenden Konzentrationen praktisch nicht mehr möglich ist. Dadurch wird der praktisch nutzbare Assay-Bereich für quantitative Bestimmungen weitere eingeschränkt.

Eine literaturbekannte Methode, um den Hook-Effekt zu erkennen, sieht eine Doppelbestimmung der Probe mit zwei unterschiedlichen Probenverdünnungen vor, so daß im Falle einer sehr hohen Konzentration die stärker verdünnte Probe ein höheres Meßsignal erzeugt (Schulze und Schwick, Prot. Biol. Fluids 5 (1958), 15). Weiterhin hat man versucht, durch Analyse der Kinetik der Antikörper-Antigenreaktion den Hook-Effekt zu erkennen und zu eliminieren (DE 27 24 2722, EP 0 148 463, Hoffmann et al., Clin. Chem. 30, (1984), 1499). Diese oben genannten Verfahren besitzen jedoch alle den Nachteil, daß entweder eine komplizierte Durchführung der Messung oder eine komplizierte Auswertung der Messung notwendig ist.

Die Durchführung der sequenziellen Assaytechnik kann in der Regel den Hook-Effekt verhindern, besitzt jedoch die schon genannten Nachteile der geringeren Empfindlichkeit und Präzision sowie längere Meßzeiten als bei einem simultanen Sandwich-Assay.

Eine bessere Möglichkeit zur Vermeidung von Fehlinterpretationen bei simultanen Sandwich-Immunoassays würde darin bestehen, die Gestalt der Eichkurve soweit zu verändern, daß das Auftreten des Hook-Effektes vermieden oder zumindest in einen höheren Konzentrationsbereich verschoben werden kann. Hierdurch würde ein größerer Assaybereich für den praktischen Test resultieren.

Es ist beschrieben worden, daß man zur Verringerung des Hook-Effektes neben den normalerweise in einem Immuntest vorhandenen hochspezifischen Antikörpern zumindestens einen weiteren Antikörper zusetzt, der eine geringe Affinität für den Analyten besitzt (US 4 595 661). Der Nachteil dieses Verfahrens besteht darin, daß für jeden nachzuweisenden Analyten zwei spezifische Antikörper mit deutlich unterschiedlichen Affinitäten für den Analyten hergestellt werden müssen.

Eine Aufgabe der vorliegenden Erfindung war es deshalb, einen Sandwich-Assay so zu verbessern, daß zum einen der Hook-Effekt zu möglichst hohen Konzentrationsbereichen verschoben werden kann und daß auf diese Weise zum anderen die Steilheit der Kurve bis zu höheren Analytkonzentrationen so hoch bleibt, daß nah beieinanderliegende Analytkonzentrationen durch ihre zugehörigen Meßsignale gut voneinander unterschieden werden können. Dadurch würde der Assaybereich für eine praktische Anwendung bis zu höheren Konzentrationen vergrößert werden können.

Gegenstand dieser Erfindung sind solche simultanen ("Ein-Schritt-") Immunoassays, bei denen mindestens einer der beiden Antikörper des Sandwichassays ein polyklonaler Antikörper ist. Die erfindungsgemäße Aufgabe wird dadurch gelöst, daß man den (oder die) polyklonalen Antikörper mit Hilfe eines affinitätschromatographischen Verfahrens aufreinigt, wobei an das Trenngel das den Antikörpern entsprechende Antigen kovalent gebunden ist.

Bei dem erfindungsgemäßen Verfahren werden die polyklonalen Antikörper über eine Chromatographiesäule aufgereinigt, die mit einem Gel gefüllt ist, an das das zum jeweiligen aufzureinigenden Antikörper komplementäre Antigen-Protein kovalent gebunden ist. Nur die zum Protein spezifischen Antikörper werden auf der Säule gebunden, während alle unspezifischen Antikörper ungehindert aus der Säule eluiert werden. Die spezifischen, an der Säule gebundenen Antikörper können dann beispielsweise durch Wechsel des pH-Wertes von der Säule eluiert, aufkonzentriert und weiterverarbeitet werden. Ein solches Verfahren zur affinitätschromatographischen Reinigung von Antikörpern ist in der Literatur ausführlich beschrieben (P. D. G. Dean, W. S. Johnson und F. A. Middle (Eds.) Affinity Chromatography. A Practical Approach, IRL Press (1985)).

Ein bevorzugtes Beispiel der vorliegenden Erfindung beinhaltet einen simultanen Sandwich-Immunoassay, der als Festphasenantikörper einen monoklonalen Antikörper enthält und als signalerzeugenden Antikörper einen polyklonalen Antikörper. Dieser polyklonale Antikörper ist gemaß dieses hier beschriebenen erfindungsgemäßen Verfahrens über eine Chromatographiesäule aufgereinigt, die ein Gel enthält, an welches das entsprechende Antigen kovalent gebunden ist.

Ein ganz besonders bevorzugtes Beispiel dieser vorliegenden Erfindung beinhaltet einen simultanen Sandwich-Immunoassay zur quantitativen Bestimmung von beta-2-Mikroglobulin. Hier wird ein monoklonaler Fängerantikörper und ein polyklonaler signalerzeugender Antikörper verwendet. Der polyklonale Antikörper wird aufgrund des in dieser Anmeldung beschriebenen Verfahrens über eine Chromatographiesäule gereinigt, die mit einem Gel gefüllt ist, an welches reines beta-2-Mikroglobulin kovalent gebunden ist. Durch dieses Verfahren erreicht man, daß der Hook-Peak zu höheren Analytkonzentrationen verschoben wird und die Eichkurve für einen größeren Konzentrationsbereich geeignet wird, im Vergleich zu signalgebenden Antikörpern, die nicht affinitätschromatographisch gereinigt wurden.

Die im folgenden aufgeführten Beispiele verdeutlichen die Erfindung anhand eines Verfahrens zur quantitativen Bestimmung von beta-2-Mikroglobulin, ohne daß diese Beispiele in irgendeiner Weise die gesamten Ansprüche dieser Anmeldung begrenzen.

### Beispiel 1

Der Sandwich-Immunoassay zur quantitativen Bestimmung von beta-2-Mikroglobulin wurde auf dem automatischen Analysengerät Immuno 1 (Bayer Diagnostics, München) durchgeführt. Es wird ein monoklonaler Antikörper A gegen beta-2-Mikroglobulin benutzt sowie ein polyklonaler Antikörper B gegen beta-2-Mikroglobulin aus Ziegenserum.

Der monoklonale Antikörper A wird als Fängerantikörper benutzt, indem er nach literaturbekannten Verfahren (z. B. S. S. Wong, Chemistry of Protein Conjugation and Cross-Linking, CRC Press, 1991) mit Fluoresceinisothiocyanat (FITC) gelabelt wird.

Der polyklonale Antikörper B wird aus Ziegenserum durch Ammoniumsulfatfällung und anschließend über eine Chromatographiesäule gereinigt, an dessen Trenngel (BioRad Affi-Prep 10 Affinity Chromatography Support) beta-2-Mikroglobulin kovalent gebunden ist. Nach erfolgter Isolierung wird dieser affinitätsgereinigte Antikörper nach literaturbekannten Verfahren (z. B. S. S. Wong, Chemistry of Protein Conjugation and Cross-Linking, CRC Press, 1991) mit dem Enzym "Alkalische Phosphatase" gekoppelt. Dieses Konjugat dient als signalerzeugendes Antikörperreagenz.

Beide Antikörperkonjugate werden in wässriger, proteinhaltiger Pufferlösung als Nachweisreagenz für den Test auf dem Immuno 1-Automaten verwendet.

Zunächst werden auf dem Gerät beide Antikörper und die Probe 21 min bei 37 °C inkubiert, um den Sandwich-Komplex zu bilden. Danach werden magnetische Partikel zugegeben und weitere 8 min bei 37 °C inkubiert. Die magnetischen Partikel sind mit Antikörpern gegen Fluorescein beschichtet und binden an das Fluorescein des Fängerantikörpers des Immunkomplexes. Nach Abtrennen des Immunkomplexes, Waschen und Zugabe von p-Nitrophenolphosphat katalysiert das Enzym Alkalische Phosphatase, welches an den zweiten Antikörper des Immunkomplexes gebunden ist, die Bildung von farbigem p-Nitrophenolat. Die in den ersten Minuten gebildete Menge an Farbstoff ist der Analytkonzentration proportional.

Zum Vergleich wurde der polyklonale Antikörper B aus Ziegenserum über eine Säule gereinigt, die Protein A enthält, und danach wie oben beschrieben mit Alkalischer Phosphatase gekoppelt. Wie oben beschrieben, wird er als signalgebender Antikörper zusammen mit dem fluoresceinierten monoklonalen Antikörper A zur Bestimmung von beta-2-Mikroglobulin auf dem Immuno 1 benutzt.

Das Versuchsergebnis zeigt, daß nur dann eine quantitative Bestimmung im Bereich von 0-20 mg/l beta-2-Mikroglobulin möglich ist, wenn der polyklonale Antikörper vor dem Koppeln an Alkalische Phosphatase über eine Trennsäule gereinigt wird, an dessen Trenngel beta-2-Mikroglobulin kovalent gebunden ist. Gewinnt man den Antikörper dagegen aus Ziegenserum durch Protein A-Chromatographie, erhält man eine Eichkurve, die schon bei einer Analytkonzentration von etwa 8 mg/l eine Sättigung erreicht und zur quantitativen Bestimmung von beta-2-Mikroglobulinkonzentrationen im Bereich von 8-20 mg/l nicht mehr in der Lage ist. Eine Erhöhung der Protein A - gereinigten Antikörperkonjugatkonzentration im Reagenz führt nicht zur Verbesserung des Versuchsergebnisses.

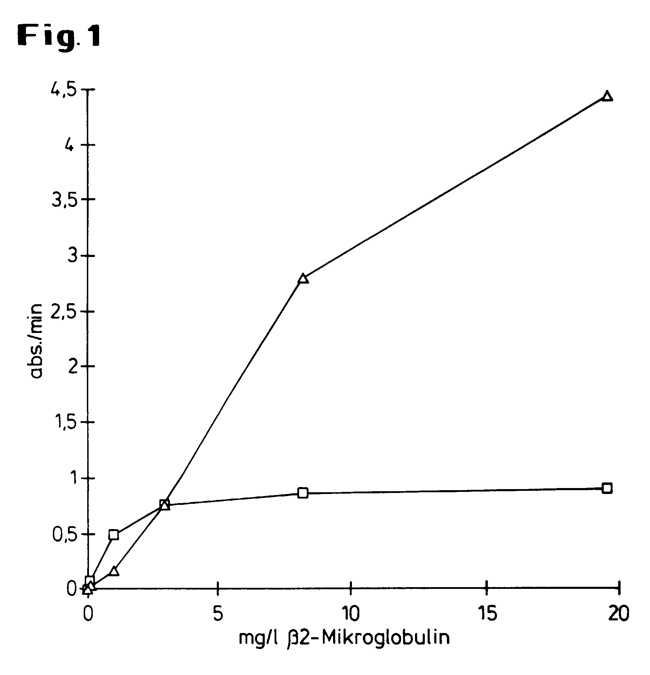

Fig. 1 zeigt das Ergebnis in graphischer Form. Es wurden Eichkurven mit 6 Standardkontrollen erstellt. Diese Kontrollen haben die Werte 0, 0.1, 1.0, 3.0, 8.0 und 20.0 mg/l. Diejenige Eichkurve, deren Meßpunkte durch ein Dreieck wiedergegeben werden, beschreibt den Verlauf der Eichkurve des Antikörperpaares, welches den affinitätsgereinigten polyklonalen Antikörper als signalgebenden Antikörper enthält. Die Eichkurve, deren Meßpunkte durch eine Raute wiedergegeben sind, beschreibt den Verlauf des Antikörperpaares, welches den Protein A-gereinigten polyklonalen Antikörper als signalgebenden Antikörpers erhält. Auf der x-Achse sind die Analytkonzentrationen aufgetragen, die y-Achse gibt die vom Analysengerät gemessenen Absorptionswertänderung pro Minute an.

### Beispiel 2

Als Fängerantikörper wird der fluoresceinierte Antikörper A benutzt, wie unter Beispiel 1 beschrieben.

Der polyklonale Antikörper C wird aus Ziegenserum durch Fällen mit Ammoniumsulfat und anschließende affinitätschromatographische Reinigung über eine Chromatographiesäule gewonnen, die mit einem Gel gefüllt ist, an das beta-2-Mikroglobulin kovalent gebunden ist.

Zum Vergleich wird derselbe polyklonale Antikörper nach Fällung aus Ziegenserum über eine Protein A - Säule gereinigt.

Die Konjugation an Alkalische Phosphatase erfolgt für beide Antikörperfraktionen wie unter Beispiel 1 beschrieben. Beide Konjugate werden als signalgebender Antikörper benutzt.

Die Durchführung des Immunoassays erfolgt wie unter Beispiel 1 beschrieben.

Das Versuchsergebnis zeigt, daß nur der affinitätsgereinigte polyklonale Antikörper als signalgebendes Antikörperkonjugat eine Eichkurve erzeugt, die im Bereich 0-20 mg/l quantitativ beta-2-Mikroglobulin bestimmen kann. Setzt man hingegen den polyklonalen Antikörper in Protein A - gereinigter Form ein, erreicht die Eichkurve sehr schnell Sättigung und ist zur Bestimmung von Analytkkonzentrationen von 10-20 mg/l nicht mehr in der Lage. Eine Erhöhung der Konzentration des Protein A-gereinigten Antikörperkonjugats verbessert das Versuchsergebnis nicht.

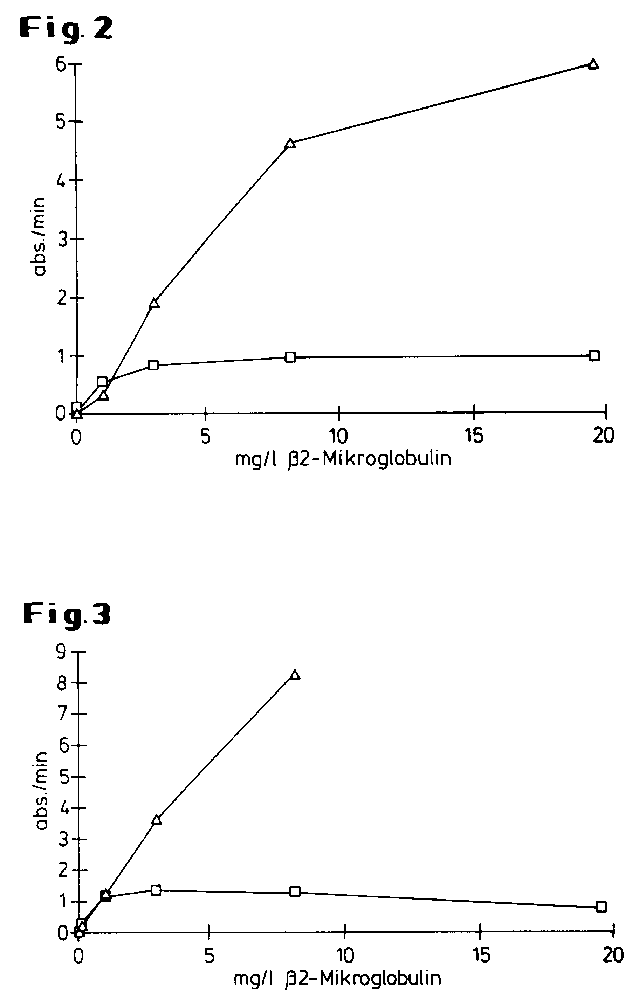

Fig. 2 zeigt das Ergebnis in graphischer Form. Es wurden Eichkurven mit 6 Standardkontrollen erstellt. Diese Kontrollen haben die Werte 0, 0.1, 1.0, 3.0, 8.0 und 20.0 mg/l. Diejenige Eichkurve, deren Meßpunkte durch ein Dreieck wiedergegeben werden, beschreibt den Verlauf der Eichkurve des Antikörperpaares, welches den affinitätsgereinigten polyklonalen Antikörper als signalgebenden Antikörper enthält. Die Eichkurve, deren Meßpunkte durch eine Raute wiedergegeben sind, beschreibt den Verlauf des Antikörperpaares, welches den Protein A-gereinigten polyklonalen Antikörper als signalgebenden Antikörpers erhält. Auf der x-Achse sind die Analytkonzentrationen aufgetragen, die y-Achse gibt die vom Analysengerät gemessenen Absorptionswertänderung pro Minute an.

### Beispiel 3

Als Fängerantikörper wird der monoklonale Antikörper D verwendet und wie in Beispiel 1 beschrieben mit FITC konjugiert. Als signalgebender Antikörper wird das Alkalische Phosphatase-Konjugat des polyklonalen Antikörpers B verwendet. Wie in Beispiel 1 beschrieben, wird der Antikörper einmal affinitätschromatographisch und einmal über Protein A gereinigt.

Die Durchführung des Immunoassays erfolgt wie in Beispiel 1 beschrieben.

Das Versuchsergebnis zeigt, daß die Antikörperkombination mit dem affinitätsgereinigten signalgebenden Antikörper eine Eichkurve ergibt, die stark ansteigt.

Setzt man dagegen den signalgebenden Antikörper in Protein A-gereinigter Form ein, erkennt man in der Eichkurve einen deutlichen Hook-Effekt mit einem Hook-Peak schon bei etwa 3-5 mg/l Analytkonzentration.

Fig. 3 zeigt das Ergebnis in graphischer Form. Es wurden Eichkurven mit 6 Standardkontrollen erstellt. Diese Kontrollen haben die Werte 0, 0.1, 1.0, 3.0, 8.0 und 20.0 mg/l. Diejenige Eichkurve, deren Meßpunkte durch ein Dreieck wiedergegeben werden, beschreibt den Verlauf der Eichkurve des Antikörperpaares, welches den affinitätsgereinigten polyklonalen Antikörper als signalgebenden Antikörper enthält. Die Eichkurve, deren Meßpunkte durch eine Raute wiedergegeben sind, beschreibt den Verlauf des Antikörperpaares, welches den Protein A-gereinigten polyklonalen Antikörper als signalgebenden Antikörpers erhält. Auf der x-Achse sind die Analytkonzentrationen aufgetragen, die y-Achse gibt die vom Analysengerät gemessenen Absorptionswertänderung pro Minute an.

Der Wert für die 20 mg/l-Kontrolle im Fall des affinitätsgereinigten signalgebenden Antikörpers ist höher als für die 8 mg/l-Kontrolle. Da er jedoch außerhalb des Meßbereiches lag und kein exakter Wert ermittelt werden konnte, wurde er im Diagramm nicht abgebildet.

### Beispiel 4

Als Fängerantikörper wird der monoklonale Antikörper D verwendet und wie in Beispiel 1 beschrieben mit FITC konjugiert. Als signalgebender Antikörper wird das Alkalische Phosphatase-Konjugat des polyklonalen Antikörpers C verwendet. Wie in Beispiel 2 beschrieben, wird der Antikörper einmal affinitätschromatographisch und einmal über Protein A gereinigt.

Die Durchführung des Immunoassays erfolgt wie in Beispiel 1 beschrieben.

Das Versuchsergebnis zeigt, daß die Antikörperkombination mit dem affinitätsgereinigten signalgebenden Antikörper eine Eichkurve ergibt, die erst bei hohen Konzentrationen langsam eine Sättigung erreicht.

Setzt man dagegen den signalgebenden Antikörper in Protein A-gereinigter Form ein, erkennt man in der Eichkurve einen deutlichen Hook-Effekt mit einem Hook-Peak schon bei etwa 3-5 mg/l Analytkonzentration.

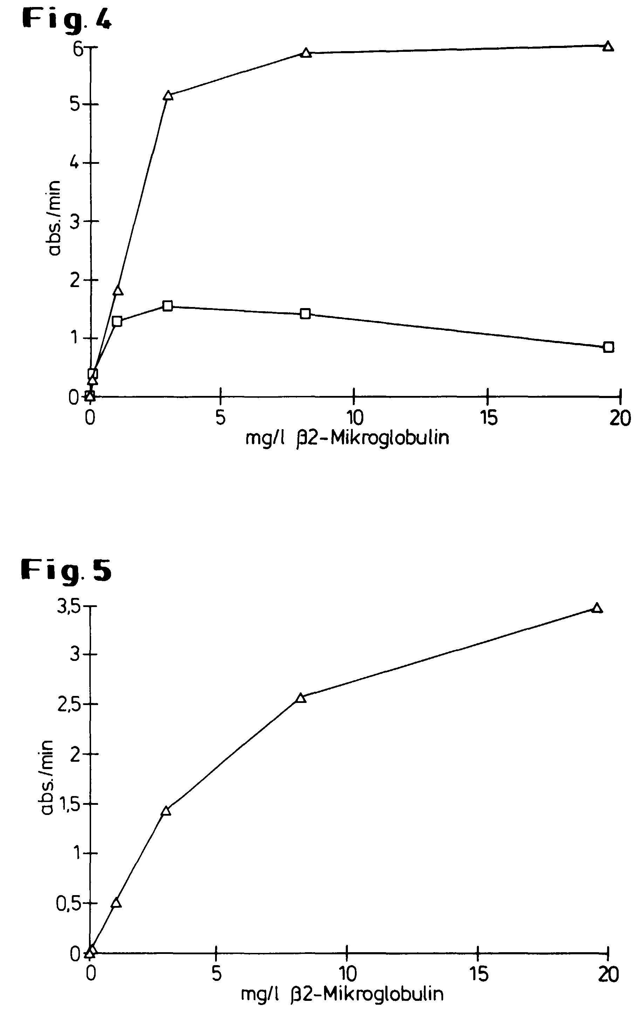

Fig. 4 zeigt das Ergebnis in graphischer Form. Es wurden Eichkurven mit 6 Standardkontrollen erstellt. Diese Kontrollen haben die Werte 0, 0.1, 1.0, 3.0, 8.0 und 20.0 mg/l. Diejenige Eichkurve, deren Meßpunkte durch ein Dreieck wiedergegeben werden, beschreibt den Verlauf der Eichkurve des Antikörperpaares, welches den affinitätsgereinigten polyklonalen Antikörper als signalgebenden Antikörper enthält. Die Eichkurve, deren Meßpunkte durch eine Raute wiedergegeben sind, beschreibt den Verlauf des Antikörperpaares, welches den Protein A-gereinigten polyklonalen Antikörper als signalgebenden Antikörpers erhält. Auf der x-Achse sind die Analytkonzentrationen aufgetragen, die y-Achse gibt die vom Analysengerät gemessenen Absorptionswertänderung pro Minute an.

### Beispiel 5

Fängerantikörper: monoklonaler Antikörper A, mit FITC konjugiert wie unter Beispiel 1 beschrieben.

Signalgebender Antikörper: polyklonaler Antikörper E, affinitätsgereinigt über eine mit beta-2-Mikroglobulin beladene Säule und mit Alkalischer Phosphatase konjugiert wie unter Beispiel 1 beschrieben.

Versuchsdurchführung wie für Beispiel 1 beschrieben.

Die erzeugte Eichkurve ermöglicht die quantitative Bestimmung von beta-2-Mikroglobulin bis zu Konzentrationen von 20 mg/l.

Fig. 5 zeigt das Ergebnis in graphischer Form. Es wurden Eichkurven mit 6 Standardkontrollen erstellt. Diese Kontrollen haben die Werte 0, 0.1, 1.0, 3.0, 8.0 und 20.0 mg/l. Das gezeigte Diagramm beschreibt den Verlauf der Eichkurve des Antikörperpaares, welches den affinitätsgereinigten polyklonalen Antikörper als signalgebenden Antikörper enthält. Auf der x-Achse sind die Analytkonzentrationen aufgetragen, die y-Achse gibt die vom Analysengerät gemessenen Absorptionswertänderung pro Minute an.

## Patentansprüche

1. Testkit für einen simultanen Sandwichimmunoassay enthaltend polyklonale Antikörper, dadurch gekennzeichnet, daß diese Antikörper am Antigen affinitätschromatographisch gereinigt wurden.

2. Testkit gemäß Anspruch 1, worin der polyklonale Antikörper der signalerzeugende Antikörper ist.

3. Testkit gemäß Anspruch 2, worin der polyklonale Antikörper durch Farbstoffe, Nuklide oder Enzyme markiert ist.

4. Testkit gemäß den Ansprüchen 1 bis 3 zum Nachweis von beta-2-Mikroglobulin.

5. Verwendung von am Antigen affinitätschromatographisch gereinigten polyklonalen Antikörpern zur Herstellung von Immunoassays.

6. Verfahren zur Vergrößerung des Assaybereiches bei einem simultanen Sandwichimmunoassay (Ein-Schritt-Assay) durch Verwendung von polyklonalen Antikörpern, die gegen das jeweilige Antigen affinitätschromatographisch gereinigt sind.

7. Verfahren nach Anspruch 6, dadurch gekennzeichnet, daß mindestens ein polyklonaler Antikörper verwendet und gegen das Antigen affinitätschromatographisch gereinigt wird.

8. Verfahren nach den Ansprüchen 6 und 7, dadurch gekennzeichnet, daß der Fängerantikörper einen monoklonalen Antikörper darstellt, und der signalgebende zweite Antikörper ein polyklonaler Antikörper ist, der gegen das Antigen affinitätschromatographisch gereinigt ist.

9. Verfahren gemäß den Ansprüchen 6 - 8, wobei das Antigen beta-2-Mikroglobulin ist.

10. Verfahren zum Nachweis von beta-2-Mikroglobulin mit einem simultanen Sandwich-Immunoassay, wobei der Fängerantikörper einen monoklonalen fluoresceinierten Antikörper darstellt, und der signalgebende Antikörper einen polyklonalen Antikörper darstellt, der am beta-2-Mikroglobulin affinitätschromatographisch gereinigt ist und mit Alkalischer Phosphatase gekoppelt ist, und wobei die Festphasenimmobilisierung durch magnetische Partikel erfolgt, an die anti-Fluorescein-Antikörper gebunden sind.
